# EUROPÄISCHE PATENTSCHRIFT

(11) **EP 3 367 155 B1**
(45) Veröffentlichungstag und Bekanntmachung des Hinweises auf die Patenterteilung: **17.11.2021**
(21) Anmeldenummer: 18157846.9
(22) Anmeldetag: 21.02.2018
(51) Int. Cl.: G02B 23/24, A61B 1/00

(54) **VORRICHTUNG ZUR ERFASSUNG EINES STEREOBILDS**
DEVICE FOR RECORDING A STEREO IMAGE
DISPOSITIF DE DÉTECTION D'UNE IMAGE STÉRÉO

(30) Priorität: 23.02.2017 DE 102017103721
(43) Veröffentlichungstag der Anmeldung: 29.08.2018
(73) Patentinhaber: Karl Storz SE & Co. KG, 78532 Tuttlingen (DE)
(72) Erfinder: HENI, Andreas, 78532 Tuttlingen (DE); KUPFERSCHMID, Markus, 78532 Tuttlingen (DE); NATUSCH, Lawrence, 78532 Tuttlingen (DE); ULMSCHNEIDER, Daniel, 78532 Tuttlingen (DE)

(56) Entgegenhaltungen:
- US-A1- 2016 242 634

## Beschreibung

Die vorliegende Erfindung ist auf eine Vorrichtung zur Erfassung eines Stereobilds bezogen, die ein Schwenken oder Rotieren der Blickrichtung ermöglicht.

In DE 10 2010 041 857 A1 ist ein Stereoendoskop mit einer seitlichen oder schrägen Blickrichtung, d. h. mit einer Blickrichtung, die von einer zentralen 0°-Blickrichtung abweicht, beschrieben. Die seitliche Blickrichtung ist durch distale Ablenkelemente bestimmt (Absatz [0024]). Die Änderung der Blickrichtung erfolgt durch Drehung der distalen Ablenkelemente (Absatz [0024]). Auch ein geneigtes distales Sichtfenster, hinter dem die distalen Ablenkelemente angeordnet sind, wird rotiert (Absatz [0026]). Die distalen Ablenkelemente vollziehen gleichläufige Bewegungen, wobei während der Drehung der axiale Abstand zwischen dem Bildsensor und dem Ablenkelement sich nicht verändert (Absatz [0027]). Jeder Bildsensor ist zusammen mit dem zugeordneten Ablenkelement in axialer Richtung beweglich (Absatz [0029]). In US 2013/0310648 A1 (auch veröffentlicht als US 9,204,787 B2) ist ein Stereoendoskop mit schwenkbarer Blickrichtung beschrieben. Das Endoskop weist zwei optische Kanäle auf, die synchron um ihre Achsen rotiert und gleichzeitig synchron und gegensätzlich parallel zu ihren Achsen verschoben werden.

Eine Aufgabe der vorliegenden Erfindung besteht darin, eine verbesserte Vorrichtung zur Erfassung eines Stereobilds zu schaffen, die insbesondere eine Rotation der Blickrichtung auf einem Kegelmantel ohne eine Rotation der Basis zulässt.

Diese Aufgabe wird durch die Gegenstände der unabhängigen Ansprüche gelöst.

Weiterbildungen sind in den abhängigen Ansprüchen angegeben.

Eine Vorrichtung zur Erfassung eines Stereobilds umfasst ein erstes Objektiv zur Erzeugung eines ersten Bilds, das dafür vorgesehen ist, mit einem ersten Auge eines Betrachters betrachtet zu werden, ein zweites Objektiv zur Erzeugung eines zweiten Bilds, das dafür vorgesehen ist, mit einem zweiten Auge eines Betrachters betrachtet zu werden, eine erste Blickrichtungseinrichtung, die um eine erste Rotationsachse rotierbar und dem ersten Objektiv zugeordnet ist, und eine zweite Blickrichtungseinrichtung, die um eine zweite Rotationsachse rotierbar und dem zweiten Objektiv zugeordnet ist, wobei durch simultane Rotation der ersten Blickrichtungseinrichtung um die erste Rotationsachse und der zweiten Blickrichtungseinrichtung um die zweite Rotationsachse die Blickrichtung der Vorrichtung rotierbar ist, wobei das erste Objektiv oder ein Teil des ersten Objektivs translatorisch bewegbar ist, und wobei ein Kurvengetriebe vorgesehen und ausgebildet ist, um eine Rotation der ersten Blickrichtungseinrichtung mit einer translatorischen Bewegung des ersten Objektivs oder eines Teils des ersten Objektivs zu koppeln.

Eine Vorrichtung zur Erfassung eines Stereobilds umfasst ein erstes Objektiv zur Erzeugung eines ersten Bilds, das dafür vorgesehen ist, mit einem ersten Auge eines Betrachters betrachtet zu werden, ein zweites Objektiv zur Erzeugung eines zweiten Bilds, das dafür vorgesehen ist, mit einem rechten Auge eines Betrachters betrachtet zu werden, eine erste Blickrichtungseinrichtung, die um eine erste Rotationsachse rotierbar und dem ersten Objektiv zugeordnet ist, eine zweite Blickrichtungseinrichtung, die um eine zweite Rotationsachse rotierbar und dem zweiten Objektiv zugeordnet ist, einen ersten Bildsensor zum Erfassen des ersten Bilds und einen zweiten Bildsensor zum Erfassen des zweiten Bilds, wobei durch simultane Rotation der ersten Blickrichtungseinrichtung um die erste Rotationsachse und der zweiten Blickrichtungseinrichtung um die zweite Rotationsachse die Blickrichtung der Vorrichtung rotierbar ist, wobei das erste Objektiv oder ein Teil des ersten Objektivs translatorisch bewegbar ist, und wobei ein Kurvengetriebe vorgesehen und ausgebildet ist, um eine Rotation der ersten Blickrichtungseinrichtung mit einer translatorischen Bewegung des ersten Objektivs oder eines Teils des ersten Objektivs zu koppeln.

Die Vorrichtung ist insbesondere ein Stereoendoskop oder ein Stereoexoskop für medizinische oder nicht medizinische Anwendungen.

Das erste Objektiv und das zweite Objektiv sind insbesondere baugleich. Das von dem ersten Objektiv erzeugte erste Bild und das von dem zweiten Objektiv erzeugte zweite Bild können durch die zugeordneten Bildsensoren in analoge oder digitale Bildsignale gewandelt werden, die verstärkt, gefiltert, aufbereitet, bearbeitet, gespeichert werden und/oder eine Wiedergabe an einer Anzeigevorrichtung steuern können. Die Bildsensoren können unmittelbar proximal der zugeordneten Objektive angeordnet sein, um die durch die Objektive erzeugten Bilder unmittelbar zu erfassen. Alternativ kann jedem Objektiv ein Stablinsensystem oder ein anderes Relaislinsensystem oder ein geordnetes Bündel von Lichtleitfasern zugeordnet sein, um eine optische Übertragung des von dem Objektiv erzeugten Bilds beispielsweise zu einem proximalen Ende der Vorrichtung zu ermöglichen.

Beide Blickrichtungseinrichtungen sind insbesondere baugleich. Jede Blickrichtungseinrichtung umfasst insbesondere eine oder mehrere reflektierende ebene oder gekrümmte Flächen, beispielsweise innerhalb eines oder an einem Prisma oder zwischen mehreren Prismen.

Die erste Blickrichtungseinrichtung und das erste Objektiv sind Teil eines ersten Strahlengangs. Die im folgenden als erste optische Achse bezeichnete optische Achse des ersten Strahlengangs weist durch die erste Blickrichtungseinrichtung einen oder mehrere Knicke auf, so dass die erste optische Achse distal und proximal der Blickrichtungseinrichtung unterschiedliche Richtungen aufweist. Licht brechende Grenzflächen an oder zwischen Linsen des ersten Objektivs sind insbesondere rotationssymmetrisch zu der ersten optischen Achse. Im folgenden wird zwischen einem distalen Abschnitt und einem proximalen Abschnitt der ersten optischen Achse unterschieden. Der distale Abschnitt der ersten optischen Achse ist der distal der ersten Blickrichtungseinrichtung gelegene Abschnitt der ersten optischen Achse. Der proximale Abschnitt der ersten optischen Achse ist der proximal der ersten Blickrichtungseinrichtung gelegene Abschnitt der ersten optischen Achse.

Die zweite Blickrichtungseinrichtung und das zweite Objektiv sind Teil eines zweiten Strahlengangs. Die im Folgenden als zweite optische Achse bezeichnete optische Achse des zweiten Strahlengangs weist durch die erste Blickrichtungseinrichtung einen oder mehrere Knicke auf, so dass die zweite optische Achse distal und proximal der Blickrichtungseinrichtung unterschiedliche Richtungen aufweist. Licht brechende Grenzflächen an oder zwischen Linsen des zweiten Objektivs sind insbesondere rotationssymmetrisch zu der zweiten optischen Achse. Im folgenden wird zwischen einem distalen Abschnitt und einem proximalen Abschnitt der zweiten optischen Achse unterschieden. Der distale Abschnitt der zweiten optischen Achse ist der distal der zweiten Blickrichtungseinrichtung gelegene Abschnitt der zweiten optischen Achse. Der proximale Abschnitt der zweiten optischen Achse ist der proximal der zweiten Blickrichtungseinrichtung gelegene Abschnitt der zweiten optischen Achse.

Die erste Blickrichtungseinrichtung kann distal des ersten Objektivs und separat von dem ersten Objektiv angeordnet sein. In diesem Fall sind insbesondere alle Licht brechende Grenzflächen des ersten Objektivs rotationssymmetrisch zu dem proximalen Abschnitt der ersten optischen Achse. Dabei kann die Vorrichtung vorgesehen und ausgebildet sein, um das erste Objektiv abhängig von der rotatorischen Position der ersten Blickrichtungseinrichtung lediglich translatorisch zu bewegen (beispielsweise zusammen mit einem zugeordneten Bildsensor, um die Bildweite konstant zu halten), jedoch nicht zu rotieren.

Alternativ kann die Vorrichtung ausgebildet sein, um das erste Objektiv zusammen mit der ersten Blickrichtungseinrichtung zu rotieren. Dabei können die erste Blickrichtungseinrichtung und das erste Objektiv insbesondere starr miteinander verbunden sein oder ein gemeinsames Bauteil bilden und/oder teilweise oder vollständig integriert sein.

Alternativ kann das erste Objektiv teilweise oder vollständig distal der ersten Blickrichtungseinrichtung vorgesehen und angeordnet sein. Dabei sind insbesondere alle oder ein Teil der Licht brechenden Grenzflächen des ersten Objektivs rotationssymmetrisch zu dem distalen Abschnitt der ersten optischen Achse. In diesem Fall werden die erste Blickrichtungseinrichtung und das erste Objektiv zusammen um die erste Rotationsachse rotiert und dabei translatorisch bewegt.

Die erste Rotationsachse entspricht insbesondere dem proximalen Abschnitt der ersten optischen Achse. Die Richtung der translatorischen Bewegbarkeit des ersten Objektivs und optional der ersten Blickrichtungseinrichtung ist insbesondere parallel zu der ersten Rotationsachse und damit zu dem proximalen Abschnitt der ersten optischen Achse.

Für die zweite Blickrichtungseinrichtung und den zweiten Strahlengang gilt insbesondere weitgehend oder vollständig Entsprechendes.

Insbesondere kann auch das zweite Objektiv translatorisch bewegbar sein. In diesem Fall kann ein weiteres Kurvengetriebe vorgesehen und ausgebildet sein, um eine Rotation der zweiten Blickrichtungseinrichtung mit einer translatorischen Bewegung des zweiten Objektivs zu koppeln. Das Kurvengetriebe und das weitere Kurvengetriebe sind insbesondere so ausgebildet, dass die translatorische Bewegung des ersten Objektivs und die translatorische Bewegung des zweiten Objektivs jederzeit gegenläufig sind. Alternativ kann lediglich ein Kurvengetriebe vorgesehen sein, das die Rotation der ersten Blickrichtungseinrichtung mit der translatorischen Bewegung des ersten Objektivs koppelt, während das zweite Objektiv unbewegt oder zumindest nicht translatorisch (d. h. insbesondere nicht oder nur rotatorisch) bewegt ist.

Die Blickrichtung des ersten Strahlengangs ist die Richtung, in der ein weit entfernter Gegenstand bezogen auf das distale Ende der Vorrichtung liegt, wenn er auf die Mitte des ersten Bilds abgebildet wird. Die Blickrichtung des zweiten Strahlengangs ist die Richtung, in der ein weit entfernter Gegenstand bezogen auf das distale Ende der Vorrichtung liegt, wenn er auf die Mitte des zweiten Bilds abgebildet wird. Die Blickrichtung der Vorrichtung ist die Richtung, in der ein weit entfernter Gegenstand oder ein Gegenstand in einem vorbestimmten Abstand bezogen auf das distale Ende der Vorrichtung liegt, wenn er auf die Mitte des ersten Bilds und auf die Mitte des zweiten Bilds abgebildet wird.

Die erste Blickrichtungseinrichtung und die zweite Blickrichtungseinrichtung sind insbesondere so miteinander rotatorisch gekoppelt, dass jederzeit die Blickrichtungen beider Blickrichtungseinrichtungen parallel sind und einer einzigen gemeinsamen Blickrichtung der Vorrichtung entsprechen. Alternativ können die erste Blickrichtungseinrichtung und die zweite Blickrichtungseinrichtung beispielsweise so gekoppelt sein, dass die Blickrichtungen beider Strahlengänge einander jederzeit in einem vorbestimmten Abstand vom distalen Ende der Vorrichtung schneiden.

Beispielsweise schneidet der proximale Abschnitt der ersten optischen Achse den ersten Bildsensor in dessen Mitte oder in der Mitte des durch den ersten Bildsensor erfassten Bilds, und der proximale Abschnitt der zweiten optischen Achse schneidet den zweiten Bildsensor in dessen Mitte oder in der Mitte des durch den zweiten Bildsensor erfassten Bilds. In diesem Fall ist insbesondere die Richtung des distalen Abschnitts der ersten optischen Achse die Blickrichtung des ersten Strahlengangs, und die Richtung des distalen Abschnitts der zweiten optischen Achse ist die Blickrichtung des zweiten Strahlengangs. Die Bewegung der ersten Blickrichtungseinrichtung und die Bewegung der zweiten Blickrichtungseinrichtung sind insbesondere so gekoppelt, dass die distalen Abschnitte der ersten optischen Achse und der zweiten optischen Achse und damit die erste Blickrichtung und die zweite Blickrichtung jederzeit parallel sind. Alternativ können die Bewegung der ersten Blickrichtungseinrichtung und die Bewegung der zweiten Blickrichtungseinrichtung beispielsweise so gekoppelt sein, dass die distalen Abschnitte der ersten optischen Achse und der zweiten optischen Achse einander jederzeit in einem vorbestimmten Abstand schneiden.

Die Blickrichtung der Vorrichtung ist insbesondere auf einem Kegelmantel rotierbar, wobei die Symmetrieachse des Kegelmantels der Symmetrieachse des distalen Endes eines Schafts der Vorrichtung entspricht oder zu dieser parallel ist. Der halbe Öffnungswinkel des Kegelmantels entspricht dem Winkel, um den jede Blickrichtungseinrichtung die Blickrichtung bzw. die optische Achse des zugeordneten Strahlengangs bricht. Dieser Winkel kann für beide Blickrichtungseinrichtungen jeweils konstant, d. h. unveränderbar, oder für beide Blickrichtungseinrichtungen simultan veränderbar sein.

Auch bei einer herkömmlichen Vorrichtung zur Erfassung eines Stereobilds, bei der die Blickrichtung nicht parallel zu der Längsachse eines Schafts der Vorrichtung ist, kann die Blickrichtung auf einem Kegelmantel rotiert werden, indem die Vorrichtung um die Längsachse des Schafts rotiert wird. Dabei werden aber auch die Bildsensoren und die Basis, d. h. die Richtung der Verbindungsstrecke der Mitten der beiden Bildsensoren, rotiert. Deshalb rotiert ein mit dieser herkömmlichen Vorrichtung erfasstes Stereobild ebenfalls, was rasch zu einem Verlust der Orientierung führt und die Führung von medizinischen Instrumenten zumindest extrem erschweren kann. Zwar könnten die beiden Bilder jeweils digital zurück rotiert werden, so dass im wiedergegeben Stereobild oben und unten, links und rechts erhalten bleiben. Aber die Rotation der Basis wäre nicht eliminierbar und würde zu einer Zerstörung der räumlichen Wahrnehmung führen.

Im Gegensatz zu einer solchen herkömmlichen Vorrichtung ermöglicht die Rotation der Blickrichtungseinrichtungen der hier beschriebenen Vorrichtung eine Rotation der Blickrichtungen ohne eine Rotation der Bildsensoren und ohne eine Rotation der Basis.

Das Kurvengetriebe ermöglicht eine einfache und zuverlässige Kopplung der Rotation der ersten Blickrichtungseinrichtung mit einer translatorischen Bewegung des ersten Objektivs. Damit kann erreicht werden, dass unabhängig von der eingestellten Blickrichtung für Gegenstände in der eingestellten Blickrichtung die Gegenstandsweite und damit auch der Abbildungsmaßstab für beide Strahlengänge gleich oder im Wesentlichen gleich ist.

Bei einer Vorrichtung, wie sie hier beschrieben ist, ist insbesondere das Kurvengetriebe oder ein weiteres Kurvengetriebe vorgesehen und ausgebildet, um eine Rotation der ersten Blickrichtungseinrichtung mit einer translatorischen Bewegung der ersten Blickrichtungseinrichtung in der Richtung, in der das erste Objektiv oder ein Teil des ersten Objektivs bewegbar ist, zu koppeln.

Wenn das erste Objektiv und die erste Blickrichtungseinrichtung integriert sind oder ein gemeinsames Bauteil bilden oder starr miteinander verbunden sind oder so miteinander verbunden sind, dass sie relativ zueinander nur um die erste Rotationsachse rotierbar sind, kann das Kurvengetriebe eine simultane translatorische Bewegung der ersten Blickrichtungseinrichtung und des ersten Objektivs bewirken. Wenn die erste Blickrichtungseinrichtung und das erste Objektiv nicht miteinander verbunden sind, können ein erstes Kurvengetriebe die Rotation der ersten Blickrichtungseinrichtung mit der translatorischen Bewegung des ersten Objektivs und ein zweites Kurvengetriebe die Rotation der ersten Blickrichtungseinrichtung mit der translatorischen Bewegung der ersten Blickrichtungseinrichtung koppeln.

Eine Vorrichtung, wie sie hier beschrieben ist, umfasst insbesondere ferner ein weiteres Kurvengetriebe, das vorgesehen und ausgebildet ist, um eine Rotation der ersten Blickrichtungseinrichtung mit einer translatorischen Bewegung der ersten Blickrichtungseinrichtung in der Richtung, in der das erste Objektiv bewegbar ist, zu koppeln, wobei das weitere Kurvengetriebe vorgesehen und ausgebildet ist, um die erste Blickrichtungseinrichtung innerhalb eines Bereichs translatorisch zu bewegen, der kleiner ist als der Bereich, innerhalb dessen das Kurvengetriebe das erste Objektiv translatorisch bewegt.

Insbesondere dann, wenn die Brechkraft der ersten Blickrichtungseinrichtung Null beträgt oder deutlich kleiner ist als die übriger Bestandteile des ersten Strahlengangs ist (d. h. die Brennweite der ersten Blickrichtungseinrichtung unendlich oder sehr viel größer als die Brennweite der übrigen Bestandteile des ersten Strahlengangs ist), hat die translatorische Position der ersten Blickrichtungseinrichtung keinen oder nur einen kleinen Einfluss auf die Position des ersten Bilds. In diesem Fall kann die erste Blickrichtungseinrichtung weniger als das erste Objektiv oder sogar nicht bewegt werden ohne die Position des ersten Bilds zu beeinflussen.

Die erste Blickrichtungseinrichtung innerhalb eines kleineren Bereichs und damit weniger zu bewegen als das erste Objektiv oder sogar gar nicht zu bewegen kann eine teilweise Abschattung durch den Rand eines Fensterbauteils am distalen Ende der Vorrichtung verringern oder verhindern.

Die erste Blickrichtungseinrichtung innerhalb eines kleineren Bereichs und damit weniger zu bewegen als das erste Objektiv kann eine von der Blickrichtung abhängige Variation der Breite der Basis, d. h. des Abstands der distalen Abschnitte der optischen Achsen verhindern. Bei einem Stereoendoskop oder Stereoexoskop oder einer anderen Vorrichtung zur Erfassung eines Stereobilds mit zwei geraden und parallelen Strahlengängen ist die Basis die gerade Strecke zwischen den Mitten der beiden Bildsensoren und der Abstand der optischen Achsen. Im hier vorliegenden Fall einer Vorrichtung zur Erfassung eines Stereobilds mit abgewinkelten oder abgeknickten Strahlengängen ist die Basis der Abstand der distalen Abschnitte der optischen Achsen - sofern die optischen Achsen dort parallel sind. Im Falle nicht paralleler optischer Achsen ist die Basis der Abstand der distalen Abschnitte der optischen Achsen an den Eintrittspupillen der beiden Strahlengänge.

Bei einer Vorrichtung, wie sie hier beschrieben ist, ist insbesondere das Kurvengetriebe oder ein weiteres Kurvengetriebe vorgesehen und ausgebildet, um eine Rotation der ersten Blickrichtungseinrichtung mit einer translatorischen Bewegung des dem ersten Objektiv zugeordneten ersten Bildsensors oder einer Lichteintrittsfläche einer Bildübertragungseinrichtung zu koppeln.

Das erste Objektiv und ein dem ersten Objektiv zugeordneter erster Bildsensor oder das erste Objektiv und eine Lichteintrittsfläche einer dem ersten Objektiv zugeordneten Bildübertragungseinrichtung können mechanisch starr miteinander verbunden oder so miteinander verbunden sein, dass das erste Objektiv relativ zu dem zugeordneten Bildsensor oder zu der Lichteintrittsfläche der zugeordneten Bildübertragungseinrichtung lediglich rotiert werden kann. In diesem Fall ist ein einziges Kurvengetriebe ausreichend, um auch eine translatorische Bewegung des dem ersten Objektiv zugeordneten ersten Bildsensors oder der Lichteintrittsfläche der dem ersten Objektiv zugeordneten Bildübertragungseinrichtung mit einer Rotation der ersten Blickrichtungseinrichtung zu koppeln.

Wenn das erste Objektiv nicht mit dem zugeordneten ersten Bildsensor oder der Lichteintrittsfläche einer zugeordneten Bildübertragungseinrichtung unmittelbar mechanisch gekoppelt ist, kann ein erstes Kurvengetriebe eine translatorische Bewegung des ersten Objektivs mit einer Rotation der ersten Blickrichtungseinrichtung koppeln und ein zweites Kurvengetriebe eine translatorische Bewegung des dem ersten Objektiv zugeordneten ersten Bildsensors oder einer Lichteintrittsfläche einer dem ersten Objektiv zugeordneten Bildübertragungseinrichtung mit einer Rotation der ersten Blickrichtungseinrichtung koppeln.

Indem die Bildweite, also der Abstand zwischen dem ersten Objektiv und dem ersten Bildsensor, konstant gehalten wird, bleibt die Fokussierung konstant, d. h. der Abstand, in dem Objekte liegen, die scharf auf den ersten Bildsensor abgebildet werden, bleibt unabhängig von der eingestellten Blickrichtung unverändert.

Bei einer Vorrichtung, wie sie hier beschrieben ist, umfasst das erste Objektiv insbesondere einen ersten Teil und einen zweiten Teil, wobei die Vorrichtung so ausgebildet ist, dass entweder nur die translatorische Bewegung des ersten Teils mit der Rotation der ersten Blickrichtungseinrichtung gekoppelt ist, oder dass eine Rotation der ersten Blickrichtungseinrichtung mit unterschiedlichen translatorischen Bewegungen des ersten Teils und des zweiten Teils des ersten Objektivs einhergeht.

Insbesondere ist der erste Teil unmittelbar oder mittelbar (über Kurvengetriebe) so mit der ersten Blickrichtungseinrichtung gekoppelt, dass eine Rotation der ersten Blickrichtungseinrichtung mit synchronen translatorischen Bewegungen der Blickrichtungseinrichtung und des ersten Teils des ersten Objektivs einhergeht. Dabei kann der zweite Teil des ersten Objektivs - insbesondere zusammen mit dem ersten Bildsensor - ruhen oder eine andere translatorische Bewegung ausführen, beispielsweise mit einer geringeren Geschwindigkeit und innerhalb eines kürzeren Pfads. Insbesondere sind der Ort des zweiten Teils des ersten Objektivs und der Ort des ersten Bildsensors unabhängig von der rotatorischen Position der ersten Blickrichtungseinrichtung.

Der erste Teil des ersten Objektivs ist insbesondere distal des zweiten Teils angeordnet. Der erste Teil und der zweite Teil des ersten Objektivs sind insbesondere so ausgebildet, dass Licht, das von einem Objekt, das letztlich scharf auf den ersten Bildsensor abgebildet wird, ausgeht, zwischen dem ersten Teil und dem zweiten Teil des Objektivs parallel oder im Wesentlichen parallel verläuft.

Eine Vorrichtung, wie sie hier beschrieben ist, umfasst insbesondere ferner eine Antriebswelle, die mit der ersten Blickrichtungseinrichtung und mit der zweiten Blickrichtungseinrichtung rotatorisch gekoppelt ist.

Die rotatorische Kopplung der Blickrichtungseinrichtungen mit einer Antriebswelle ermöglicht die Rotation der Blickrichtungseinrichtungen und damit der Blickrichtung der Vorrichtung durch Rotation der Antriebswelle. Die Antriebswelle ermöglicht also die mechanische Steuerung der Blickrichtung.

Die Antriebswelle und die Blickrichtungseinrichtungen sind insbesondere durch Zahnräder miteinander rotatorisch gekoppelt. Beispielsweise sind an den äußeren Umfängen von Fassungen der Blickrichtungseinrichtungen Zähne vorgesehen, die Zahnräder bilden. Zahnräder an den Blickrichtungseinrichtungen sind insbesondere gleich groß, ein Zahnrad an der Antriebswelle, das mit den Zahnrädern an den Blickrichtungseinrichtungen kämmt, kann einen kleineren oder größeren Durchmesser aufweisen, um eine Unter- oder Übersetzung zu bewirken.

Bei einer Vorrichtung, wie sie hier beschrieben ist, können die erste Blickrichtungseinrichtung und die zweite Blickrichtungseinrichtung jeweils zumindest teilweise neben der Antriebswelle angeordnet sein, während das erste Objektiv und das zweite Objektiv jeweils zumindest teilweise neben der Antriebswelle angeordnet sind.

Insbesondere sind der proximale Abschnitt der ersten optischen Achse des ersten Strahlengangs und der proximale Abschnitt der zweiten optischen Achse des zweiten Strahlengangs jeweils neben der Antriebswelle oder neben dem distalen Ende der Antriebswelle angeordnet. Die Antriebswelle ist also insbesondere keine Hohlwelle, in deren Inneren die Strahlengänge angeordnet sind. Im Fall einer Hohlwelle, innerhalb derer die Strahlengänge angeordnet sind, schrumpft der Durchmesser des für die Strahlengänge zur Verfügung stehenden Bauraums mindestens um die doppelte Wandstärke der Hohlwelle. Im Vergleich dazu kann bei einer Anordnung der Blickrichtungseinrichtungen neben der Antriebswelle oder deren distalem Ende mehr Bauraum für die Strahlengänge zur Verfügung stehen. Die Antriebswelle ist insbesondere zumindest teilweise zwischen den Blickrichtungseinrichtungen angeordnet. Bei einer Vorrichtung, wie sie hier beschrieben ist, umfasst die Antriebswelle insbesondere einen Kurventräger, der Bestandteil des Kurvengetriebes ist, oder ist mit einem Kurventräger, der Bestandteil des Kurvengetriebes ist, rotatorisch gekoppelt, wobei an der Außenseite des Kurventrägers eine Kurve, die Bestandteil des Kurvengetriebes ist, zur Kopplung einer Rotation der ersten Blickrichtungseinrichtung mit einer translatorischen Bewegung des ersten Objektivs oder eines Teils des ersten Objektivs vorgesehen ist.

Die Kurve ist beispielsweise als Nut, Steg oder Stufe an dem Kurventräger ausgebildet. Der Kurventräger kann gleichzeitig als Zahnrad zur rotatorischen Kopplung mit den Blickrichtungseinrichtungen ausgebildet sein. An dem Kurventräger können mehrere Kurven vorgesehen sein, beispielsweise je eine Kurve für jedes Objektiv (oder jeweils einen Teil jedes Objektivs) und/oder je eine Kurve für jede Blickrichtungseinrichtung und/oder je eine Kurve für jeden Bildsensor. Jede Kurve kann die Rotation des Kurventrägers - und damit mittelbar die Rotation der Blickrichtungseinrichtungen - mit einer translatorischen Bewegung eines zugeordneten Objektivs (oder eines Teils des zugeordneten Objektivs) oder einer zugeordneten Blickrichtungseinrichtung oder eines zugeordneten Bildsensors koppeln. Indem verschiedene Kurven vorgesehen sind, können beispielsweise beide Objektive (oder Teile beider Objektive) entgegengesetzt zu einander bewegt werden und/oder beide Blickrichtungseinrichtungen entgegengesetzt zu einander bewegt werden und/oder beide Bildsensoren entgegengesetzt zu einander bewegt werden. Ferner können verschieden Kurven verschiedene Steigungen oder Ganghöhen aufweisen, um beispielsweise eine Blickrichtungseinrichtung weniger stark zu bewegen als das zugeordnete Objektiv (oder einen Teil des zugeordneten Objektivs) und den zugeordneten Bildsensor.

Bei einer Vorrichtung, wie sie hier beschrieben ist, umfasst das Kurvengetriebe insbesondere eine Nut oder einen Steg oder eine Stufe an der Antriebswelle oder an einem mit der Antriebswelle rotatorisch gekoppelten Kurventräger und einen Zapfen oder einen Steg oder einen anderen Abtaster an dem ersten Objektiv oder einem Teil des ersten Objektivs oder an einem mit dem ersten Objektiv oder einem Teil des ersten Objektivs translatorisch gekoppelten Bauteil.

Bei einer Vorrichtung, wie sie hier beschrieben ist, umfasst das Kurvengetriebe insbesondere eine Nut oder einen Steg oder eine Stufe an dem ersten Objektiv oder einem Teil des ersten Objektivs oder an einem mit dem ersten Objektiv oder einem Teil des ersten Objektivs translatorisch gekoppelten Bauteil und einen Abtaster an der Antriebswelle oder an einem mit der Antriebswelle rotatorisch gekoppelten Bauteil.

Die Antriebswelle kann also gleichzeitig der Rotation der Blickrichtungseinrichtungen dienen und Bestandteil des Kurvengetriebes zur Kopplung der translatorischen Bewegung des ersten Objektivs (oder eines Teils des ersten Objektivs) mit der rotatorischen Bewegung der ersten Blickrichtungseinrichtung sein. Dies kann eine kompakte Bauweise der Vorrichtung ermöglichen.

Bei einer Vorrichtung, wie sie hier beschrieben ist, ist die Antriebswelle insbesondere zwischen dem ersten Objektiv und dem zweiten Objektiv angeordnet.

Insbesondere liegen die optischen Achsen der Objektive, die in diesem Fall die proximalen Abschnitte der optischen Achsen der Strahlengänge sind, und die Rotationsachse der Antriebswelle in einer Ebene, wobei die Abstände der Rotationsachse der Antriebswelle von den optischen Achsen der Objektive gleich sein können.

Alternativ liegt die Rotationsachse der Antriebswelle nicht in der Ebene, in der die optischen Achsen der Objektive oder die proximalen Abschnitte der optischen Achsen der Strahlengänge liegen. Dabei können trotzdem die Abstände der Rotationsachse der Antriebswelle zu den proximalen Abschnitten der optischen Achsen der Strahlengänge gleich sein. Die Antriebswelle nicht zwischen den Objektiven, sondern seitlich versetzt anzuordnen, kann Bauraum einsparen und somit mehr Bauraum für die Objektive und andere Bauteile ermöglichen.

Eine Vorrichtung, wie sie hier beschrieben ist, umfasst insbesondere ferner einen Schaft mit einem distalen Ende, wobei die äußere Mantelfläche des Schafts zumindest nahe dem distalen Ende des Schafts rotationssymmetrisch zu einer Symmetrieachse ist, wobei die erste Blickrichtungseinrichtung, die zweite Blickrichtungseinrichtung, das erste Objektiv und das zweite Objektiv in dem Schaft angeordnet sind, und wobei zumindest bei einer Blickrichtung der Vorrichtung ein proximaler Abschnitt der optischen Achse der ersten Blickrichtungseinrichtung und ein proximaler Abschnitt der optischen Achse der zweiten Blickrichtungseinrichtung nicht spiegelsymmetrisch zu einer Symmetrieebene, die die Symmetrieachse des Schafts enthält, angeordnet sind.

Die erste Blickrichtungseinrichtung, die zweite Blickrichtungseinrichtung, das erste Objektiv und das zweite Objektiv sind insbesondere nahe dem distalen Ende des Schafts in dem Schaft angeordnet. Die optischen Achsen der Blickrichtungseinrichtungen an deren proximalem Ende sind insbesondere zumindest bei einer Blickrichtung nicht gleich weit beabstandet von der Symmetrieachse der äußeren Mantelfläche des Schafts. Alternativ liegen zumindest bei einer Blickrichtung die optischen Achsen der Blickrichtungseinrichtungen an deren proximalen Enden und die Symmetrieachse der äußeren Mantelfläche des Schafts nicht in einer Ebene.

Eine derartige nicht spiegelsymmetrische Anordnung kann eine Abschattung durch einen Rand eines Fensterbauteils am distalen Ende des Schafts verhindern oder vermindern.

Eine Vorrichtung, wie sie hier beschrieben ist, umfasst insbesondere ferner eine Einrichtung zum translatorischen Bewegen der Blickrichtungseinrichtungen in Richtung orthogonal zu der Symmetrieachse des Schafts abhängig von der eingestellten Blickrichtung.

Die Blickrichtungseinrichtungen und mit ihnen zusammen insbesondere auch die Objektive und die Bildsensoren können abhängig von der eingestellten Blickrichtung entlang einer geraden Strecke verschoben oder auf Kreisbahnen oder anderen nicht-geraden Bahnen bewegt werden (jeweils bezogen auf eine Schnittebene orthogonal zu den proximalen Abschnitten der optischen Achsen und orthogonal zu der Symmetrieachse des Schafts). Vereinfacht dargestellt werden die Blickrichtungseinrichtungen und insbesondere die gesamten Strahlengänge beispielsweise nach links bewegt, wenn die Blickrichtung nach rechts geht, und nach rechts bewegt, wenn die Blickrichtung nach links geht. Optional können die Blickrichtungseinrichtungen und insbesondere die gesamten Strahlengänge nach unten bewegt werden, wenn die Blickrichtung nach oben geht, und nach oben, wenn die Blickrichtung nach unten geht. Dies kann die Abschattung durch den Rand eines Fensterbauteils vermindern oder verhindern.

Bei einer Vorrichtung, wie sie hier beschrieben ist, ist insbesondere jede der beiden Blickrichtungseinrichtungen so ausgebildet, dass bezogen auf eine Projektion parallel zu dem proximalen Abschnitt der optischen Achse der Blickrichtungseinrichtung der Schnittpunkt des distalen Abschnitts der optischen Achse der Blickrichtungseinrichtung mit der Lichteintrittsfläche der Blickrichtungseinrichtung außerhalb des Querschnitts der Blickrichtungseinrichtung an deren proximalem Ende liegt.

Anders ausgedrückt, versetzt die Blickrichtungseinrichtung die optische Achse zwischen der Lichteintrittsfläche am distalen Ende und der Lichtaustrittsfläche am proximalen Ende um mehr als den halben Durchmesser der Blickrichtungseinrichtung an deren proximalem Ende. Dieses Versetzen kann eine Abschattung durch den Rand eines transparenten Fensterbauteils am distalen Ende der Vorrichtung verhindern oder vermindern.

### Kurzbeschreibung der Figuren

Nachfolgend werden Ausführungsformen anhand der beigefügten Figuren näher erläutert. Es zeigen:
- Figur 1: eine schematische axonometrische Darstellung eines distalen Teils einer Vorrichtung zur Erfassung eines Stereobilds;
- Figur 2: eine weitere schematische Schnittdarstellung des distalen Teils der Vorrichtung aus Figur 1;
- Figur 3: eine weitere schematische Schnittdarstellung des distalen Teils der Vorrichtung aus den Figuren 1 und 2;
- Figur 4: eine weitere schematische Schnittdarstellung des distalen Teils der Vorrichtung aus den Figuren 1 bis 3;
- Figur 5: eine weitere schematische Schnittdarstellung der Vorrichtung aus den Figuren 1 bis 4;
- Figur 6: eine schematische Schnittdarstellung einer weiteren Vorrichtung zur Erfassung eines Stereobilds;
- Figur 7: eine schematische Schnittdarstellung eines distalen Teils einer weiteren Vorrichtung zur Erfassung eines Stereobilds;
- Figur 8: eine weitere schematische Schnittdarstellung der Vorrichtung aus Figur 7;
- Figur 9: eine schematische Schnittdarstellung eines distalen Teils einer weiteren Vorrichtung zur Erfassung eines Stereobilds;
- Figur 10: eine schematische Schnittdarstellung eines distalen Teils einer weiteren Vorrichtung zur Erfassung eines Stereobilds.

### Beschreibung der Ausführungsformen

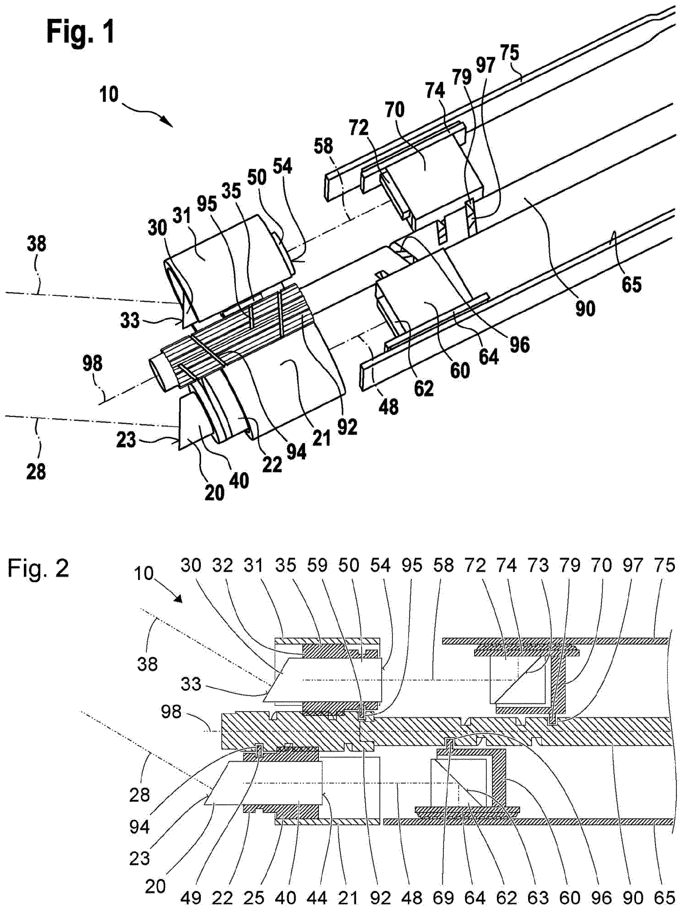

Figur 1 zeigt eine schematische axonometrische Darstellung eines distalen Teils einer Vorrichtung 10 zur Erfassung eines Stereobilds. Die Vorrichtung 10 ist insbesondere ein Stereoendoskop oder ein Stereoexoskop. Die in Figur 1 dargestellten Komponenten der Vorrichtung 10 sind beispielsweise von einem hermetischen dichten Außenschaft umschlossen, der in Figur 1 nicht dargestellt ist, und der die in Figur 1 dargestellten Komponenten vor Umwelteinflüssen schützt.

Die Vorrichtung 10 umfasst eine erste Blickrichtungseinrichtung 20 und ein erstes Objektiv 40, die bei dem dargestellten Beispiel eine Einheit bilden. Die erste Blickrichtungseinrichtung 20 und das erste Objektiv 40 sind in einer hülsenförmigen Fassung 22 gefasst und befestigt. Die Fassung 22 ist in einer ersten Führung 21 derart formschlüssig geführt, dass die Fassung 22 zusammen mit der ersten Blickrichtungseinrichtung 20 und dem ersten Objektiv 40 um die optische Achse 48 des ersten Objektivs 40 rotiert und innerhalb eines vorbestimmten Bereichs in Richtung parallel zu der optischen Achse 48 des ersten Objektivs 40 verschiebbar ist.

Das distale Ende der ersten Blickrichtungseinrichtung 20 wird durch eine Lichteintrittsfläche 23 gebildet, die gegenüber der optischen Achse 48 des ersten Objektivs 40 geneigt ist. Die erste Blickrichtungseinrichtung 20 knickt oder winkelt den durch die erste Blickrichtungseinrichtung 20 und das erste Objektiv 40 gebildeten ersten Strahlengang ab. Der erste Strahlengang und die optische Achse des ersten Strahlengangs haben deshalb an der Lichteintrittsfläche 23 der Blickrichtungseinrichtung 20 und an der Lichtaustrittsfläche der Blickrichtungseinrichtung zwei verschiedene Richtungen. Die optische Achse 48 des ersten Objektivs 40 ist die optische Achse der ersten Blickrichtungseinrichtung 20 und des ersten Strahlengangs an der Lichtaustrittsfläche der ersten Blickrichtungseinrichtung 20. Die optische Achse 28 der ersten Blickrichtungseinrichtung 20 und des ersten Strahlengangs an der Lichteintrittsfläche 23 der Blickrichtungseinrichtung 20 ist nicht parallel zu der optischen Achse 48 des ersten Objektivs 40, aber bei dem dargestellten Beispiel orthogonal zu der Lichteintrittsfläche 23 der Blickrichtungseinrichtung 20.

Die erste Blickrichtungseinrichtung 20 weist beispielsweise mehrere miteinander verkittete Prismen aus optisch transparenten Materialien auf, an deren Grenzflächen Licht reflektiert wird, insbesondere aufgrund von Totalreflexion. Diese Reflexionen bewirken, dass Licht, das parallel zu der optischen Achse 28 der ersten Blickrichtungseinrichtung an deren Lichteintrittsfläche 23 auf die Lichteintrittsfläche 23 der Blickrichtungseinrichtung 20 fällt, parallel zu der optischen Achse 48 des Objektivs 40 aus der ersten Blickrichtungseinrichtung 20 aus und in das erste Objektiv 40 eintritt.

Der Winkel zwischen der optischen Achse 28 der ersten Blickrichtungseinrichtung 20 an deren Lichteintrittsfläche 23 und der optischen Achse 48 des ersten Objektivs 40 ist bei den hier dargestellten Beispielen unveränderbar. Alternativ und abweichend von den hier dargestellten Beispielen kann der Winkel einstellbar sein.

Die Vorrichtung 10 umfasst ferner ein erstes Bildsensorbauteil 60 mit einem ersten Bildsensor 64. Bei dem dargestellten Beispiel weist das erste Bildsensorbauteil 60 ferner ein erstes Prisma 62 mit einer reflektierenden Fläche 63 auf, und der erste Bildsensor 64 ist parallel zu der optischen Achse 48 des ersten Objektivs 40 angeordnet. Der erste Bildsensor 64 ist mit dem distalen Ende einer Platine 65 verbunden. Die Platine 65 kann teilweise oder vollständig flexibel sein und/oder sich bis zu einem in Figur 1 nicht dargestellten proximalen Ende der Vorrichtung 10 erstrecken. An der Platine 65 können außer dem Bildsensor 64 weitere elektronische Bauteile zur Verstärkung, Aufbereitung, Verarbeitung von analogen oder digitalen Bildsignalen, die der erste Bildsensor 64 erzeugt, vorgesehen sein. Ferner können Leiterbahnen der ersten Platine 65 eine Übertragung eines Bildsignals zu dem in Figur 1 nicht dargestellten proximalen Ende der Vorrichtung 10 ermöglichen.

Die Vorrichtung 10 umfasst ferner eine zweite Blickrichtungseinrichtung 30 und ein zweites Objektiv 50 in einer in Figur 1 nicht sichtbaren Fassung innerhalb einer zweiten Führung 31. Die zweite Blickrichtungseinrichtung 30 mit einer Lichteintrittsfläche 33, die ihr distales Ende bildet, und einer optischen Achse 38 an der Lichteintrittsfläche 33 ist baugleich zu der ersten Blickrichtungseinrichtung 20. Das zweite Objektiv 50 mit einer Lichtaustrittsfläche 54 und einer optischen Achse 58 ist baugleich zu dem ersten Objektiv 40. Die zweite Blickrichtungseinrichtung 30 und das zweite Objektiv bilden einen zweiten Strahlengang. Die Führung 31 für die in Figur 1 nicht sichtbare Fassung der zweiten Blickrichtungseinrichtung 30 und des zweiten Objektivs 50 ist spiegelsymmetrisch oder näherungsweise spiegelsymmetrisch zu der Führung 21 der Fassung 22 der ersten Blickrichtungseinrichtung 20 und des ersten Objektivs 40 in dem in Figur 1 nicht dargestellten Außenschaft angeordnet.

Die Vorrichtung 10 umfasst ferner ein zweites Bildsensorbauteil 70 mit einem zweiten Prisma und einem zweiten Bildsensor 74, der mit dem distalen Ende einer zweiten Platine 75 verbunden ist. Das zweite Bildsensorbauteil 70 ist insbesondere baugleich zu dem ersten Bildsensorbauteil 60 und bei dem dargestellten Beispiel spiegelsymmetrisch oder im Wesentlichen spiegelsymmetrisch zu diesem angeordnet.

Die Vorrichtung 10 umfasst ferner eine Antriebswelle 90 mit einem Zahnrad 92 an dem distalen Ende der Antriebswelle 90. Die Antriebswelle 90 ist um ihre Längsachse 98 rotierbar. Die Längsachse 98 der Antriebswelle 90 ist parallel zu den optischen Achsen 48, 58 der Objektive 40, 50. Das Zahnrad 92 am distalen Ende der Antriebswelle 90 kämmt mit einem durch Zähne am äußeren Umfang der Fassung 22 der ersten Blickrichtungseinrichtung 20 und des ersten Objektivs 40 gebildeten und in Figur 1 durch die zugeordnete Führung 21 verdeckten Zahnrad und mit einem durch Zähne am äußeren Umfang der in Figur 1 im Übrigen nicht sichtbaren Fassung der zweiten Blickrichtungseinrichtung 30 und des zweiten Objektivs 50 gebildeten Zahnrad 35. Eine Rotation der Antriebswelle 90 bewirkt deshalb eine gleichzeitige Rotation der Blickrichtungseinrichtungen 20, 30 und der Objektive 40, 50 um die optischen Achsen 48, 58 der Objektive 40, 50. Die Zahnräder 35 an den äußeren Umfängen der Fassungen 22 der Blickrichtungseinrichtungen 20, 30 und der Objektive 40, 50 weisen die gleichen Durchmesser und die gleiche Anzahl Zähne auf. Jede Rotation der Antriebswelle 90 bewirkt eine gleichzeitige und gleichsinnige Rotation beider Blickrichtungseinrichtungen 20, 30 und damit der optischen Achsen 28, 38 der Blickrichtungseinrichtungen 20, 30 an deren Lichteintrittsflächen 23, 33.

An der äußeren Oberfläche der Antriebswelle 90 sind ferner mehrere Führungsnuten 94, 95, 96, 97 vorgesehen, die bei dem in Figur 1 dargestellten vereinfachten Beispiel jeweils helikal ausgebildet sind. Die erste Führungsnut 94 und die zweite Führungsnut 95 sind im Bereich des Zahnrads 92 am distalen Ende der Antriebswelle 90 ausgebildet und sind in entgegengesetzte Richtungen gewunden. Ein in Figur 1 nicht sichtbarer Abtaster in Gestalt eines mit der Fassung 22 der ersten Blickrichtungseinrichtung 20 und des ersten Objektivs 40 gekoppelten Abtaststifts greift in die erste Führungsnut 94 an der Antriebswelle 90 ein. Ein mit der Fassung der zweiten Blickrichtungseinrichtung 30 und des zweiten Objektivs 50 gekoppelter Abtaster in Gestalt eines in Figur 1 nicht sichtbaren Abtaststifts greift in die zweite Führungsnut 95 in der Antriebswelle 90 ein.

Eine dritte Führungsnut 96 und eine vierte Führungsnut 97 sind nahe den Bildsensorbauteilen 60, 70 an der Antriebswelle 90 angeordnet. Die dritte Führungsnut 96 und die vierte Führungsnut 97 sind bei dem in Figur 1 dargestellten vereinfachten Beispiel jeweils helikal und weisen entgegengesetzte Windungsrichtungen auf. Ein in Figur 1 nicht sichtbarer Abtaststift an dem ersten Bildsensorbauteil 60 greift in die dritte Führungsnut 96 ein. Ein Abtaststift 79 an dem zweiten Bildsensorbauteil 70 greift in die vierte Führungsnut 97 ein.

Die Windungsrichtungen und die Ganghöhen der ersten Führungsnut 94 und der dritten Führungsnut 96 sind gleich. Die Windungsrichtungen und Ganghöhen der zweiten Führungsnut 95 und der vierten Führungsnut 97 sind gleich. Jede Rotation der Antriebswelle 90 hat deshalb eine synchrone, d. h. gleichgerichtete und gleich schnelle translatorische Bewegung der ersten Blickrichtungseinrichtung 20, des ersten Objektivs 40 und des ersten Bildsensorbauteils 60 und eine dazu entgegengesetzte, jedoch untereinander synchrone, d. h. gleichgerichtete und gleich schnelle translatorische Bewegung der zweiten Blickrichtungseinrichtung 30, des zweiten Objektivs 50 und des zweiten Bildsensorbauteils 70 zur Folge. Wie erwähnt geht ferner durch die kämmenden Zahnräder 92, 35 jede Rotation der Antriebswelle 90 mit einer synchronen Rotation der ersten Blickrichtungseinrichtung 20, der zweiten Blickrichtungseinrichtung 30 und damit auch der optischen Achsen 28, 38 der Blickrichtungseinrichtungen 20, 30 an deren Lichteintrittsflächen 23, 33 (sowie ferner, jedoch optisch irrelevant mit einer Rotation der Objektive 40, 50) einher. Die Rotation der Blickrichtungseinrichtungen 20, 30 ist deshalb mit einer translatorischen Bewegung der Objektive 40, 50 und der Bildsensorbauteile 60, 70 gekoppelt.

Die Führungsnuten 94, 95, 96, 97 sind insbesondere so ausgebildet, dass bei jeder Blickrichtung, d. h. bei jeder Orientierung der optischen Achsen 28, 38 der Blickrichtungseinrichtungen 20, 30 an deren Lichteintrittsflächen 23, 33 die Lichteintrittsflächen 23, 33 der Blickrichtungseinrichtungen 20, 30 in einer einzigen Ebene orthogonal zu der Blickrichtung liegen. Dazu sind die Führungsnuten 94, 95, 96, 97 insbesondere abweichend von der Darstellung in Figur 1 nicht helikal im engeren Sinne (d. h. mit jeweils konstanter Steigung), sondern weisen variierende Steigungen auf. Insbesondere sind die Steigungen der Führungsnuten 94, 95, 96, 97 an den Orten, an denen die Abtaststifte 79 in die Führungsnuten 94, 95, 96, 97, eingreifen, wenn, wie in Figur 1 angedeutet, die optischen Achsen 48, 58 beider Objektive 40, 50 und die optischen Achsen 28, 38 beider Blickrichtungseinrichtungen 20, 30 an deren Lichteintrittsflächen 23, 33 in einer einzigen Ebene liegen, null.

Figur 2 zeigt eine schematische Schnittdarstellung des distalen Teils der Vorrichtung 10 aus Figur 1. Die Schnittebene der Figur 2 enthält die optischen Achsen 48, 58 der Objektive 40, 50 und die optischen Achsen 28, 38 der Blickrichtungseinrichtungen 20, 30 an deren Lichteintrittsflächen 23, 33. Schnittflächen optisch transparenter Bauteile, insbesondere der Blickrichtungseinrichtungen 20, 30, der Objektive 40, 50 und der Prismen 62, 72, sind ohne Schraffur dargestellt. Schnittflächen aller übrigen Bauteile sind schraffiert dargestellt.

In Figur 2 sind sowohl die Fassung 22 der ersten Blickrichtungseinrichtung 20 und des ersten Objektivs 40 als auch die Fassung 32 der zweiten Blickrichtungseinrichtung 30 und des zweiten Objektivs 50 und die Zahnräder 25, 35 an den Fassungen 22, 32 sichtbar. Ferner sind die Abtaststifte 49, 59 an den Fassungen 22, 32 und damit an den Blickrichtungseinrichtungen 20, 30 und den Objektiven 40, 50 sichtbar.

Um eine Rotation der Abtaststifte 49, 59 zusammen mit den Fassungen 22, 32 und damit einen Verlust des Eingriffs der Abtaststifte 49, 59 in die Führungsnuten 94, 95 zu vermeiden, ist insbesondere jeder Abtaststift 49, 59 Teil eines im Übrigen in Figur 2 nicht sichtbaren Bauteils, das einerseits in eine umfängliche Nut der zugeordneten Fassung 22, 32 und andererseits in eine Längsnut der zugeordneten Führung 21, 31 eingreift, so dass die Abtaststifte 49, 59 ausschließlich parallel zu der Zeichenebene der Figur 2 bewegt werden können.

In Figur 2 ist erkennbar, dass die Fassungen 22, 32 nicht symmetrisch zueinander ausgebildet, und die Abtaststifte 49, 59 an entgegengesetzten Enden der Fassungen 22, 32 angeordnet sind. Dadurch kann eine Überschneidung der ersten Führungsnut 94 für den ersten Abtaststift 49 an der Fassung 22 der ersten Blickrichtungseinrichtung 20 und des ersten Objektivs 40 mit der zweiten Führungsnut 95 für den zweiten Abtaststift 59 an der Fassung 32 der zweiten Blickrichtungseinrichtung 30 und des zweiten Objektivs 50 vermieden werden.

In Figur 2 sind ferner reflektierende Flächen 63, 73 in den Prismen 62, 72 sichtbar. Die reflektierenden Flächen 63, 73 sind ebenso wie die lichtempfindlichen Flächen der Bildsensoren 64, 74 orthogonal zu der Schnittebene der Figur 2.

Ferner sind in Figur 2 der dritte Abtaststift 69 an dem ersten Bildsensorbauteil 60, der in die dritte Führungsnut 96 eingreift, und der vierte Abtaststift 79 an dem zweiten Bildsensorbauteil 70, der in die vierte Führungsnut 97 an der Antriebswelle 90 eingreift, sichtbar.

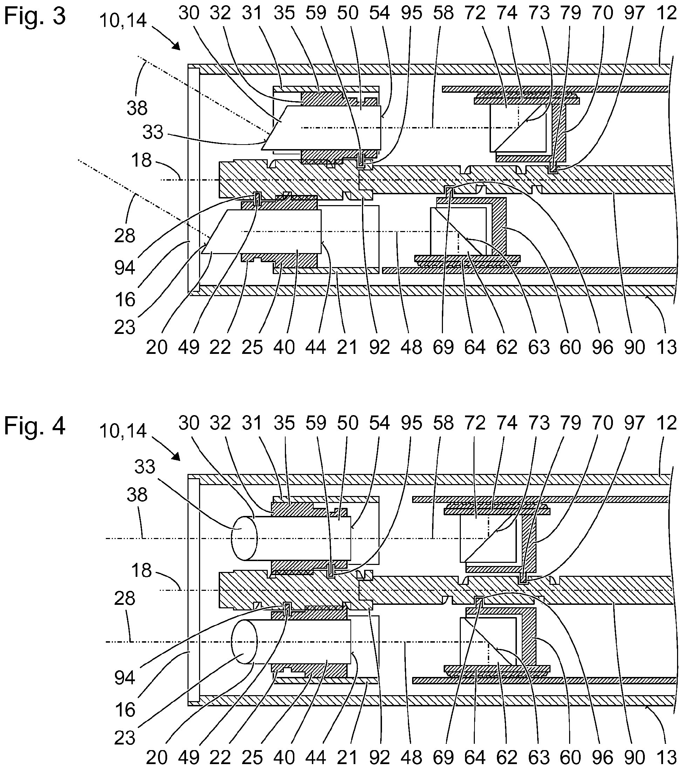

Figur 3 zeigt eine weitere schematische Darstellung eines Längsschnitts durch den distalen Teil der Vorrichtung 10 aus den Figuren 1 und 2. Die Schnittebene der Figur 3 entspricht der Schnittebene der Figur 2. Die in Figur 3 dargestellte Konfiguration entspricht der in den Figuren 1 und 2 dargestellten Konfiguration.

Die Darstellung in Figur 3 unterscheidet sich von den Darstellungen in den Figuren 1 und 2 dadurch, dass ferner ein Schaft oder Außenschaft 12 der Vorrichtung 10 dargestellt ist. Der Schaft 12 weist eine im Wesentlichen kreiszylindrische Gestalt auf, so dass seine äußere Oberfläche einen (auch als Mantelfläche bezeichneten) kreiszylindrischen Bereich 13 mit einer Symmetrieachse 18 aufweist.

Am distalen Ende 14 des Schafts 12 ist ein optisches transparentes Fensterbauteil 16 vorgesehen. Die Blickrichtungseinrichtungen 20, 30 sind unmittelbar proximal des optischen transparenten Fensterbauteils 16 angeordnet. Bei der in Figur 3 dargestellten äußerst distalen Position der ersten Blickrichtungseinrichtung 20 weist deren Lichteintrittsfläche nur einen geringen Sicherheitsabstand zu der inneren Oberfläche des optisch transparenten Fensterbauteils 16 auf.

Figur 4 zeigt eine weitere schematische Darstellung eines Schnitts durch den distalen Teil der Vorrichtung 10 aus den Figuren 1 bis 3. Die Schnittebene der Figur 4 entspricht den Schnittebenen der Figuren 2 und 3. Die Art der Darstellung in Figur 4 entspricht derjenigen der Figur 3, d. h. auch der Schaft 12 mit dem optisch transparenten Fensterbauteil 16 ist dargestellt.

Die in Figur 4 gezeigte Konfiguration unterscheidet sich von der in den Figuren 1 bis 3 dargestellten Konfigurationen dadurch, dass die Blickrichtungseinrichtungen 20, 30 (und damit auch die Objektive 40, 50) andere rotatorische und translatorische und die Bildsensorbauteile 60, 70 andere translatorische Positionen aufweisen. Bei der in Figur 4 gezeigten Konfiguration schließen die optischen Achsen 28, 38 der Blickrichtungseinrichtungen 20, 30 an deren Lichteintrittsflächen 23, 33 maximale (und untereinander gleiche) Winkel mit der Schnittebene der Figur 4 ein. Sowohl die Ebene, die die optische Achse des ersten Strahlengangs (d. h. die optische Achse 28 der ersten Blickrichtungseinrichtung 20 an deren Lichteintrittsfläche 23 und die optische Achse 48 des ersten Objektivs 40) enthält, als auch die Ebene, die die optische Achse des zweiten Strahlengangs (d. h. die optische Achse 38 der zweiten Blickrichtungseinrichtung 30 an deren Lichteintrittsfläche 33 und die optische Achse 58 des zweiten Objektivs 50) enthält, ist jeweils orthogonal zu der Schnittebene der Figur 4 und damit orthogonal zu der Ebene, in der die optischen Achsen 48, 58 der Objektive 40, 50 liegen.

Wenn von der in Figur 4 gezeigten Konfiguration ausgehend die Antriebswelle 90 in der gleichen Richtung weiter rotiert wird, wird eine Konfiguration erreicht, die zu der in Figur 3 dargestellten spiegelsymmetrisch ist.

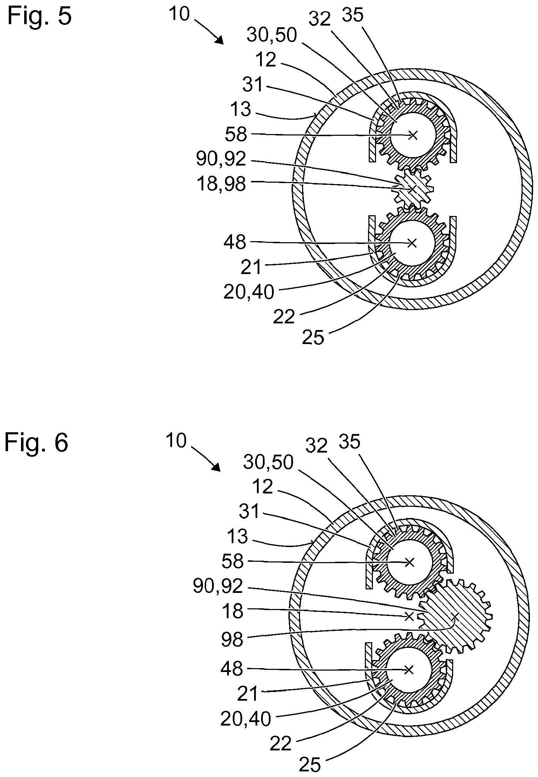

Figur 5 zeigt eine weitere schematische Darstellung eines Schnitts durch den distalen Teil der anhand der Figuren 1 bis 4 dargestellten Vorrichtung 10. Die Schnittebene der Figur 5 ist orthogonal zu den Schnittebenen der Figuren 2 bis 4, orthogonal zu der Symmetrieachse 18 des kreiszylindrischen Bereichs 13 der äußeren Oberfläche des Schafts 12 und orthogonal zu den optischen Achsen 48, 58 der Objektive 40, 50. Die Schnittebene verläuft durch das Zahnrad 92 am distalen Ende der Antriebswelle 90 und durch die Zahnräder 25, 35 an den Fassungen 22, 32 der Blickrichtungseinrichtungen 20, 30 und der Objektive 40, 50.

In Figur 5 ist der U-förmige Querschnitt jeder Führung 21, 31 erkennbar. Die Führungen 21, 31 führen die Fassungen 22, 32 spiel- und reibungsarm, wobei insbesondere die Enden der Zähne der Zahnräder 25, 35 an den inneren Oberflächen der Führungen 21, 31 anliegen. Zu der Antriebswelle 90 hin sind die Führungen 21, 31 offen, so dass das Zahnrad 92 am distalen Ende der Antriebswelle 90 mit den Zahnrädern 25, 35 an den Fassungen 22, 32 kämen kann. Figur 6 zeigt eine schematische Darstellung eines Schnitts durch den distalen Teil einer weiteren Vorrichtung 10, die in einigen Merkmalen, Eigenschaften und Funktionen der anhand der Figuren 1 bis 5 dargestellten Vorrichtung ähnelt. Die Art der Darstellung, insbesondere die Schnittebene der Figur 6 entspricht derjenigen der Figur 5.

Die in Figur 6 gezeigte Vorrichtung 10 unterscheidet sich von der anhand der Figuren 1 bis 5 dargestellten Vorrichtung insbesondere dadurch, dass die Antriebswelle 90 und das Zahnrad 92 an dem distalen Ende der Antriebswelle 90 nicht genau zwischen den Fassungen 22, 32 für die Blickrichtungseinrichtungen 20, 30 und die Objektive 40, 50 angeordnet ist. Stattdessen sind die Antriebswelle 90 und das Zahnrad 92 am distalen Ende der Antriebswelle 90 seitlich versetzt angeordnet. Die Antriebswelle 90 und das Zahnrad 92 am distalen Ende der Antriebswelle 90 rotieren deshalb nicht um die Symmetrieachse 18 des kreiszylindrischen Bereichs 13 der äußeren Oberfläche des Schafts 12.

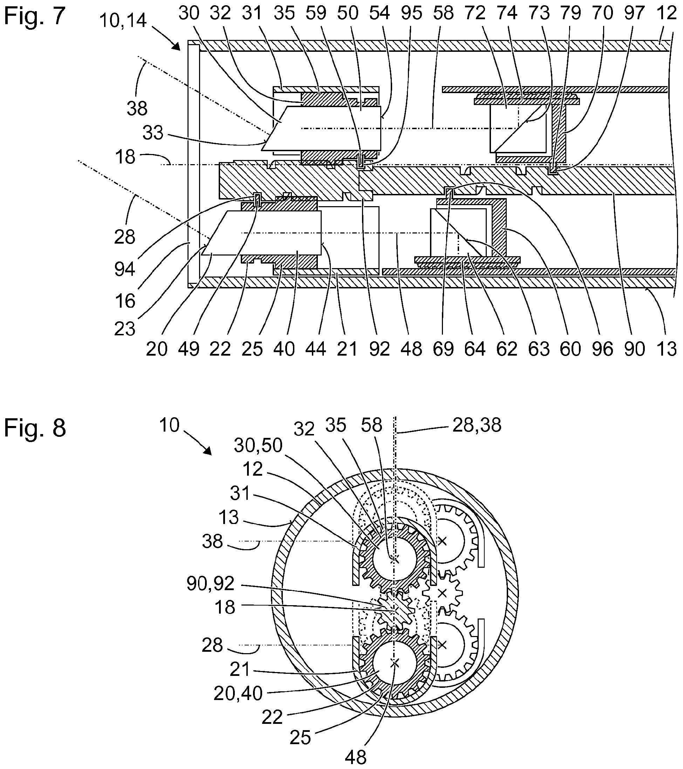

Die in Figur 6 angedeutete exzentrische Anordnung der Antriebswelle 90 kann - abweichend von der Darstellung in Figur 6 - beispielsweise größere Querschnitte der Strahlengänge, insbesondere der Blickrichtungseinrichtungen 20, 30 und der Objektive 40, 50 ermöglichen. Erfindungsgemäß befindet sich die Antriebswelle 90 noch zwischen den Objektiven 40, 50. Figur 7 zeigt eine schematische Darstellung eines Schnitts durch einen distalen Teil einer weiteren Vorrichtung 10, die in einigen Merkmalen, Eigenschaften und Funktionen den anhand der Figuren 1 bis 6 dargestellten Vorrichtungen ähnelt. Die Art der Darstellung, insbesondere die Schnittebene, entspricht derjenigen der Figuren 2 bis 4. Die in Figur 7 gezeigte Konfiguration, insbesondere die Orientierung der optischen Achsen 28, 38 der Blickrichtungseinrichtungen 20, 30 an deren Lichteintrittsflächen 23, 33 entspricht der in den Figuren 1 bis 3 gezeigten Konfiguration. Insbesondere liegen sowohl die optischen Achsen 48, 58 der Objektive 40, 50 als auch die optischen Achsen 28, 38 der Blickrichtungseinrichtungen 20, 30 an deren Lichteintrittsflächen 23, 33 in der Schnittebene der Figur 7.

Die in Figur 7 gezeigte Vorrichtung 10 unterscheidet sich von den anhand der Figuren 1 bis 6 dargestellten Vorrichtungen insbesondere durch eine asymmetrische Anordnung der Blickrichtungseinrichtungen 20, 30, der Objektive 40, 50, der Bildsensorbauteile 60, 70 und der Antriebswelle 90 bezogen auf die Symmetrieachse 18 des kreiszylindrischen Bereichs 13 der äußeren Oberfläche des Schafts 12. Diese asymmetrische Anordnung kann eine Abschattung des durch das zweite Bildsensorbauteil 70 erfassten Bilds durch den Rand des Fensterbauteils 16 verhindern oder vermindern. Die in Figur 7 gezeigte asymmetrische Anordnung kann ständig oder nur bei der in Figur 7 gezeigten Konfiguration vorliegen.

Figur 8 zeigt eine weitere schematische Darstellung eines Schnitts durch den distalen Teil der Vorrichtung 10 aus Figur 7. Die Art der Darstellung, insbesondere die Position und Orientierung der Schnittebene der Figur 8 entspricht denjenigen der Figuren 5 und 6.

In Figur 8 ist in durchgezogenen Linien und mit schraffierten Schnittflächen die in Figur 7 dargestellte Konfiguration gezeigt. Bei dieser Konfiguration zeigen die optischen Achsen 28, 38 der Blickrichtungseinrichtungen 20, 30 an deren Lichteintrittsflächen 23, 33 zu dem oberen Rand des Blattes.

In gestrichelten Linien und ohne Schraffur der Schnittflächen ist eine entgegengesetzte Konfiguration angedeutet, bei der die (nicht dargestellten) optischen Achsen der Blickrichtungseinrichtungen 20, 30 an deren Lichteintrittsflächen in entgegengesetzter Richtung zu dem unteren Rand des Blattes zeigen. Durch eine in den Figuren 7 und 8 nicht dargestellte Einrichtung können die Blickrichtungseinrichtungen 20, 30, die Objektive 40, 50, die Führungen 21, 31 und die Bildsensorbauteile 60, 70 abhängig von der eingestellten Blickrichtung zwischen diesen beiden Extrempositionen bewegt werden. Die Bewegung kann insbesondere entlang eines geraden Pfads erfolgen.

Alternativ kann die Bewegung entlang eines gekrümmten Pfads erfolgen, so dass zwischen den beiden beschriebenen Extrempositionen eine weitere, in Figur 8 in durchgezogenen Linien, jedoch ohne schraffierte Schnittflächen dargestellte Konfiguration vorliegen kann. Bei dieser Konfiguration zeigen die optischen Achsen 28, 38 der Blickrichtungseinrichtungen 20, 30 an deren Lichteintrittsflächen zu dem linken Rand des Blattes und die Blickrichtungseinrichtungen 20, 30, die Objektive 40, 50, die Führungen 21, 31 und die Bildsensorbauteile sind zu dem rechten Rand hin verschoben.

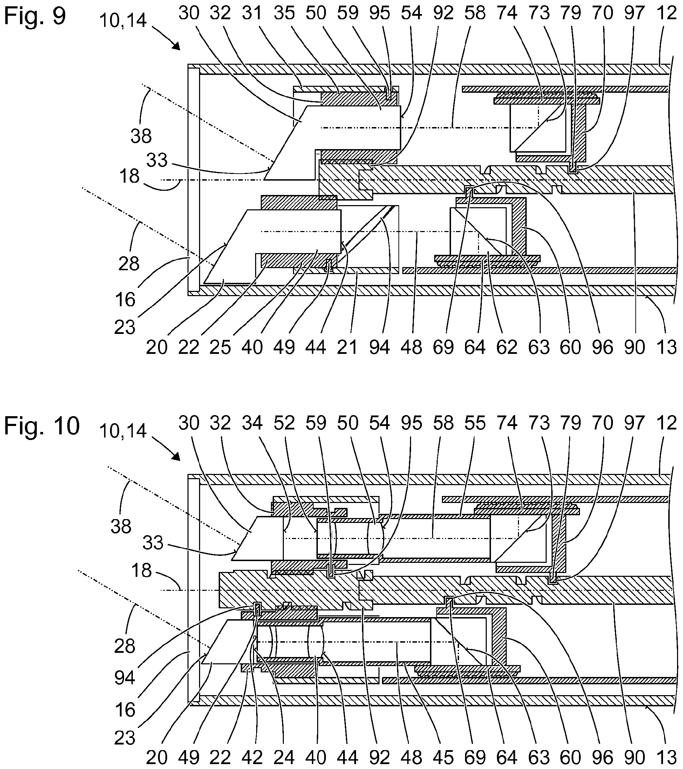

Figur 9 zeigt eine schematische Darstellung eines Schnitts durch den distalen Teil einer weiteren Vorrichtung 10, die in einigen Merkmalen, Eigenschaften und Funktionen den anhand der Figuren 1 bis 8 dargestellten, insbesondere der anhand der Figuren 1 bis 5 dargestellten Vorrichtung ähnelt. Die Schnittebene der Figur 9 entspricht der Schnittebene der Figuren 2, 3, 4 und 7, die dargestellte Konfiguration entspricht derjenigen der Figuren 1 bis 3 und 7.

Die in Figur 9 gezeigte Vorrichtung 10 unterscheidet sich von der anhand der Figuren 1 bis 5 dargestellten Vorrichtung insbesondere durch eine andere Ausgestaltung der Blickrichtungseinrichtungen 20, 30. Die Blickrichtungseinrichtungen 20, 30 der in Figur 9 gezeigten Vorrichtung sind insbesondere derart ausgebildet, dass die Schnittpunkte der optischen Achsen 28, 38 der Blickrichtungseinrichtungen 20, 30 mit deren Lichteintrittsflächen 23, 33 gegenüber den optischen Achsen 48, 58 der Objektive 40, 50 deutlich versetzt sind. Insbesondere liegt der Schnittpunkt der optischen Achse 28, 38 jeder Blickrichtungseinrichtung 20, 30 mit deren Lichteintrittsfläche 23, 33 bezogen auf eine Projektion parallel zu den optischen Achsen 48, 58 der Objektive 40, 50 außerhalb der Querschnitte der Objektive 40, 50.

Dies kann einer Abschattung - bei der in Figur 9 gezeigten Konfiguration insbesondere eine Abschattung des durch das zweite Bildsensorbauteil 70 erfassten Bilds - durch den Rand des optisch transparenten Fensterbauteils 16 verhindern oder vermindern.

Die in Figur 9 gezeigte Vorrichtung 10 unterscheidet sich von der anhand der Figuren 1 bis 5 dargestellten Vorrichtung ferner dadurch, dass Abtaststifte 49, 59 an den Fassungen 22, 32 nicht in Nuten an der Antriebswelle 90 oder dem Zahnrad 92 eingreifen, sondern in Nuten 94, 95 in den Führungen 21, 31.

Figur 10 zeigte eine schematische Darstellung eines Schnitts durch den distalen Teil einer weiteren Vorrichtung 10, die in einigen Merkmalen, Eigenschaften und Funktionen den anhand der Figuren 1 bis 9 dargestellten Vorrichtungen ähnelt. Die Art der Darstellung, insbesondere die Schnittebene der Figur 10 entspricht derjenigen der Figuren 2, 3, 4, 7 und 9. Die gezeigte Konfiguration entspricht der in den Figuren 1 bis 3, 7 und 9 gezeigten Konfiguration.

Die in Figur 10 gezeigte Vorrichtung 10 unterscheidet sich von den anhand der Figuren 1 bis 9 dargestellten Vorrichtungen insbesondere dadurch, dass die Blickrichtungseinrichtungen 20, 30 mit den zugeordneten Objektiven 40, 50 keine integralen Bauteile bilden. Vielmehr sind Lichtaustrittsflächen 24, 34 der Blickrichtungseinrichtungen 20, 30 nicht identisch mit Lichteintrittsflächen 42, 52 der Objektive 40, 50 sondern von diesen beabstandet und in Figur 10 klar unterscheidbar.

Bei dem in Figur 10 gezeigten Beispiel sind nur die erste Blickrichtungseinrichtung 20 mit der ersten Fassung 22 und nur die zweite Blickrichtungseinrichtung 30 mit der zweiten Fassung 32 starr verbunden. Das erste Objektiv 40 ist über einen ersten Tubus 45 mit dem ersten Bildsensorbauteil 60 starr verbunden. Das zweite Objektiv 50 ist über einen zweiten Tubus 55 mit dem zweiten Bildsensorbauteil 70 mechanisch starr verbunden. Die distalen Enden der Tuben 45, 55 und damit die Objektive 40, 50 können jedoch, wie in Figur 10 angedeutet, in den Fassungen 22, 32 spiel- und reibungsarm geführt sein.

Die in Figur 10 angedeutete unabhängige Steuerung der Translation der Blickrichtungseinrichtungen 20, 30 kann genutzt werden, um, wie in Figur 10 angedeutet, die Blickrichtungseinrichtungen 20, 30 weniger stark zu verschieben als die Objektive 40, 50 und die Bildsensorbauteile 60, 70. Dazu weisen insbesondere die erste Führungsnut 94 und die zweite Führungsnut 95 andere, nämlich geringere Steigungen und Ganghöhen auf als die dritte Führungsnut 96 und die vierte Führungsnut 97. Eine Folge der unterschiedlich starken Verschiebung der Blickrichtungseinrichtungen 20, 30 einerseits und der Objektive 40, 50 ist, dass der Abstand zwischen der Lichtaustrittsfläche 24 der ersten Blickrichtungseinrichtung 20 und der Lichteintrittsfläche 42 des ersten Objektivs 40 und der Abstand zwischen der Lichtaustrittsfläche 34 der zweiten Blickrichtungseinrichtung 30 und der Lichteintrittsfläche 52 des zweiten Objektivs 50 jeweils nicht konstant sind, sondern abhängig von der der Blickrichtung variieren. Bei der in Figur 10 angedeuteten Blickrichtung ist der Abstand zwischen der Lichtaustrittsfläche 24 der ersten Blickrichtungseinrichtung 20 und der Lichteintrittsfläche 42 des ersten Objektivs 40 verschieden von dem Abstand zwischen der Lichtaustrittsfläche 34 der zweiten Blickrichtungseinrichtung 30 und der Lichteintrittsfläche 52 des zweiten Objektivs 50.

Die unabhängige Steuerung der translatorischen Bewegung der Blickrichtungseinrichtungen 20, 30 kann genutzt werden, um eine Variation des Abstands der optischen Achsen 28, 38 der Blickrichtungseinrichtungen 20, 30 an deren Lichteintrittsflächen 23, 33 bei der Rotation der Blickrichtung zu verhindern. Gleichzeitig kann die Abschattung durch den Rand des optisch transparenten Fensterbauteils 16 verhindert oder vermindert werden.

Abweichend von der Darstellung in Figur 10 können nur ein Teil des ersten Objektivs 40 mit dem ersten Tubus 45 (und damit letztlich auch mit dem ersten Bildsensorbauteil 60) und nur ein Teil des zweiten Objektivs 50 mit dem zweiten Tubus 55 (und damit letztlich auch mit dem zweiten Bildsensorbauteil 70) mechanisch starr verbunden sein. In diesem Fall sind insbesondere der Rest des ersten Objektivs 40 mit der ersten Blickrichtungseinrichtung 20 mechanisch gekoppelt oder verbunden oder integriert und der Rest des zweiten Objektivs 50 mit der zweiten Blickrichtungseinrichtung 300 mechanisch gekoppelt oder verbunden oder integriert. Die Teilung der Objektive erfolgt insbesondere jeweils an einer Stelle, an der das von einem (letztlich scharf abgebildeten) Gegenstand ausgehende Licht parallel ist, so dass eine Relativbewegung der Teile jedes Objektivs keinen Einfluss auf die Abbildung, insbesondere auf die Schärfe der Abbildung hat.

Merkmale der anhand der Figuren 1 bis 10 dargestellten Vorrichtungen sind miteinander kombinierbar. Beispielsweise kann der anhand der Figur 9 angedeutete starke Versatz der optischen Achsen 28, 38 der Blickrichtungseinrichtungen 20, 30 an deren Lichteintrittsflächen 23, 33 gegenüber den optischen Achsen 48, 58 der Objektive 40, 50 mit der in Figur 10 angedeuteten unabhängigen Steuerung der Translation der Blickrichtungseinrichtungen 20, 30 kombiniert werden, um die Abschattung durch den Rand des optisch transparenten Fensterbauteils 16 weiter zu mindern.

### Bezugszeichen

- **10**: **Vorrichtung zur Erfassung eines Stereobilds**
- 12: Schaft der Vorrichtung 10
- 13: kreiszylindrischer Bereich der äußeren Oberfläche des Schafts 12
- 14: distales Ende des Schafts 12
- 16: optisch transparentes Fensterbauteil am distalen Ende 14 des Schafts 12
- 18: Symmetrieachse des kreiszylindrischen Bereichs 13
- **20**: **erste Blickrichtungseinrichtung der Vorrichtung 10**
- 21: erste Führung für die erste Blickrichtungseinrichtung 20 und das erste Objektiv 40
- 22: Fassung für die erste Blickrichtungseinrichtung 20 und das erste Objektiv 40
- 23: Lichteintrittsfläche am distalen Ende der ersten Blickrichtungseinrichtung 20
- 24: Lichtaustrittsfläche an dem proximalen Ende der ersten Blickrichtungseinrichtung 20
- 25: Zahnrad an der ersten Blickrichtungseinrichtung 20
- 28: optische Achse der ersten Blickrichtungseinrichtung 20 an deren Lichteintrittsfläche 23 und Blickrichtung der ersten Blickrichtungseinrichtung 20
- **30**: **zweite Blickrichtungseinrichtung der Vorrichtung 10**
- 31: zweite Führung für die zweite Blickrichtungseinrichtung 30 und das zweite Objektiv 50
- 32: Fassung für die zweite Blickrichtungseinrichtung 30 und das zweite Objektiv 50
- 33: Lichteintrittsfläche am distalen Ende der zweiten Blickrichtungseinrichtung 30
- 34: Lichtaustrittsfläche an dem proximalen Ende der zweiten Blickrichtungseinrichtung 30
- 35: Zahnrad an der zweiten Blickrichtungseinrichtung 30
- 38: optische Achse der zweiten Blickrichtungseinrichtung 30 an deren Lichteintrittsfläche 33 und Blickrichtung der zweiten Blickrichtungseinrichtung 30
- **40**: **erstes Objektiv der Vorrichtung 10**
- 42: Lichteintrittsfläche am distalen Ende des ersten Objektivs 40
- 44: Lichtaustrittsfläche an dem proximalen Ende des ersten Objektivs 40
- 45: erster Tubus
- 48: optische Achse des ersten Objektivs 40 und Rotationsachse der ersten Blickrichtungseinrichtung 20 und Translationsrichtung des ersten Objektivs 40
- 49: erste Abtaststift an dem ersten Objektiv 40
- **50**: **zweites Objektiv der Vorrichtung 10**
- 52: Lichteintrittsfläche am distalen Ende des zweiten Objektivs 50
- 54: Lichtaustrittsfläche an dem proximalen Ende des zweiten Objektivs 50
- 55: zweiter Tubus
- 58: optische Achse des zweiten Objektivs 50 und Rotationsachse der zweiten Blickrichtungseinrichtung 30 und Translationsrichtung des zweiten Objektivs 50
- 59: zweiter Abtaststift an dem zweiten Objektiv 50
- **60**: **erstes Bildsensorbauteil der Vorrichtung 10**
- 62: erstes Prisma des ersten Bildsensorbauteils 60
- 63: reflektierende Fläche des ersten Prismas 62
- 64: erster Bildsensor des ersten Bildsensorbauteils 60
- 65: erste Platine, mit deren distalem Ende der erste Bildsensor 64 verbunden ist
- 69: dritter Abtaststift an dem ersten Bildsensorbauteil 60
- **70**: **zweites Bildsensorbauteil der Vorrichtung 10**
- 72: zweites Prisma des zweiten Bildsensorbauteils 70
- 73: reflektierende Fläche des zweiten Prismas 72
- 74: zweiter Bildsensor des zweiten Bildsensorbauteils 70
- 75: zweite Platine, mit deren distalem Ende der zweite Bildsensor 74 verbunden ist
- 79: vierter Abtaststift an dem zweiten Bildsensorbauteil 70
- **90**: **Antriebswelle der Vorrichtung 10**
- 92: Zahnrad an der Antriebswelle 90
- 94: erste Führungsnut an der Antriebswelle 90 für den ersten Abtaststift 49 an dem ersten Objektiv 40
- 95: zweite Führungsnut an der Antriebswelle 90 für den zweiten Abtaststift 59 an dem zweiten Objektiv 50
- 96: dritte Führungsnut an der Antriebswelle 90 für den dritten Abtaststift 69 an dem ersten Bildsensorbauteil 60
- 97: vierte Führungsnut an der Antriebswelle 90 für den vierten Abtaststift 79 an dem zweiten Bildsensorbauteil 70

## Patentansprüche

1. **Vorrichtung** (10) **zur Erfassung eines Stereobilds,** mit:
einem **ersten Objektiv** (40) zur Erzeugung eines ersten Bilds, das dafür vorgesehen ist, mit einem ersten Auge eines Betrachters betrachtet zu werden;
einem **zweiten Objektiv** (50) zur Erzeugung eines zweiten Bilds, das dafür vorgesehen ist, mit einem zweiten Auge eines Betrachters betrachtet zu werden;
einer **ersten Blickrichtungseinrichtung** (20), die um eine erste Rotationsachse (48) **rotierbar** und dem ersten Objektiv (40) zugeordnet ist;
einer **zweiten Blickrichtungseinrichtung** (30), die um eine zweite Rotationsachse (58) **rotierbar** und dem zweiten Objektiv (50) zugeordnet ist,
einem **ersten Bildsensor** (64) zum Erfassen des ersten Bilds;
einem **zweiten Bildsensor** (74) zum Erfassen des zweiten Bilds; und
einem Kurvengetriebe (49, 94) mit einer Antriebswelle (90);
wobei durch simultane Rotation der ersten Blickrichtungseinrichtung (20) um die erste Rotationsachse (48) und der zweiten Blickrichtungseinrichtung (30) um die zweite Rotationsachse (58) die Blickrichtung (28, 38) der Vorrichtung (10) rotierbar ist,
wobei das erste Objektiv (40) oder ein Teil des ersten Objektivs (40) translatorisch bewegbar ist,
wobei das **Kurvengetriebe** (49, 94) ausgebildet ist, um eine Rotation der ersten Blickrichtungseinrichtung (20) mit einer translatorischen Bewegung des ersten Objektivs (40) oder eines Teils des ersten Objektivs (40) zu koppeln,
und wobei die **Antriebswelle** (90) mit der ersten Blickrichtungseinrichtung (20) und mit der zweiten Blickrichtungseinrichtung (30) rotatorisch gekoppelt und **zwischen** dem ersten Objektiv (40) und dem zweiten Objektiv (50) angeordnet ist.

2. Vorrichtung (10) nach dem vorangehenden Anspruch, bei der das Kurvengetriebe (49, 94) oder ein weiteres Kurvengetriebe (69, 96) vorgesehen und ausgebildet ist, um eine Rotation der ersten Blickrichtungseinrichtung (20) mit einer translatorischen **Bewegung der ersten Blickrichtungseinrichtung** (20) in der Richtung (48), in der das erste Objektiv (40) oder ein Teil des ersten Objektivs (40) bewegbar ist, zu koppeln.

3. Vorrichtung (10) nach einem der vorangehenden Ansprüche, bei der das Kurvengetriebe oder ein weiteres Kurvengetriebe (69, 96) vorgesehen und ausgebildet ist, um eine Rotation der ersten Blickrichtungseinrichtung (20) mit einer translatorischen **Bewegung des** dem ersten Objektiv (40) zugeordneten ersten **Bildsensors** (64) oder einer Lichteintrittsfläche einer Bildübertragungseinrichtung zu koppeln.

4. Vorrichtung (10) nach einem der vorangehenden Ansprüche, bei der das erste Objektiv (40) einen ersten Teil und einen zweiten Teil umfasst, wobei die Vorrichtung so ausgebildet ist, dass entweder nur die translatorische Bewegung des ersten Teils mit der Rotation der ersten Blickrichtungseinrichtung gekoppelt ist, oder dass eine Rotation der ersten Blickrichtungseinrichtung mit unterschiedlichen translatorischen Bewegungen des ersten Teils und des zweiten Teils des ersten Objektivs (40) einher geht.

5. Vorrichtung (10) nach einem der vorangehenden Ansprüche, bei der die erste Blickrichtungseinrichtung (20) und die zweite Blickrichtungseinrichtung (30) jeweils zumindest teilweise neben der Antriebswelle (90) angeordnet sind.

6. Vorrichtung (10) nach einem der vorangehenden Ansprüche, bei der die Antriebswelle (90) einen Kurventräger, der Bestandteil des Kurvengetriebes ist, umfasst oder mit einem Kurventräger, der Bestandteil des Kurvengetriebes ist, rotatorisch gekoppelt ist,
an der Außenseite des Kurventrägers eine Kurve (94), die Bestandteil des Kurvengetriebes ist, zur Kopplung einer Rotation der ersten Blickrichtungseinrichtung (20) mit einer translatorischen Bewegung des ersten Objektivs (40) oder eines Teils des ersten Objektivs (40) vorgesehen ist.

7. Vorrichtung (10) nach einem der vorangehenden Ansprüche, bei der das Kurvengetriebe eine **Nut** (94, 96) oder einen Steg oder eine Stufe an der Antriebswelle (90) oder an einem mit der Antriebswelle (90) rotatorisch gekoppelten Kurventräger (92) und einen Zapfen oder einen Steg oder einen anderen Abtaster an dem ersten Objektiv (40) oder einem Teil des ersten Objektivs (40) oder an einem mit dem ersten Objektiv (40) oder einem Teil des ersten Objektivs (40) translatorisch gekoppelten Bauteil (22) umfasst.

8. Vorrichtung (10) nach einem der vorangehenden Ansprüche ohne Anspruch 7, bei der das Kurvengetriebe eine **Nut** oder einen Steg oder eine Stufe an dem ersten Objektiv (40) oder einem Teil des ersten Objektivs (40) oder an einem mit dem ersten Objektiv (40) oder einem Teil des ersten Objektivs (40) translatorisch gekoppelten Bauteil (22) und einen Abtaster an der Antriebswelle (90) oder an einem mit der Antriebswelle (90) rotatorisch gekoppelten Bauteil (25) umfasst.

9. Vorrichtung (10) nach einem der vorangehenden Ansprüche, ferner mit:
einem **Schaft** (12) mit einem distalen Ende (14),
wobei die äußere Mantelfläche (13) des Schafts (12) zumindest nahe dem distalen Ende (14) des Schafts (12) **rotationssymmetrisch** zu einer Symmetrieachse (18) ist,
wobei die erste Blickrichtungseinrichtung (20), die zweite Blickrichtungseinrichtung (30), das erste Objektiv (40) und das zweite Objektiv (50), in dem Schaft (12) angeordnet sind,
wobei zumindest bei einer Blickrichtung (28) der Vorrichtung (10) ein proximaler Abschnitt (48) der optischen Achse der ersten Blickrichtungseinrichtung (20) und ein proximaler Abschnitt (58) der optischen Achse der zweiten Blickrichtungseinrichtung (30) **nicht spiegelsymmetrisch** zu einer Symmetrieebene, die die Symmetrieachse (18) des Schafts (12) enthält, angeordnet sind.

10. Vorrichtung (10) nach dem vorangehenden Anspruch, ferner mit:
einer Einrichtung zum translatorischen Bewegen der Blickrichtungseinrichtungen (20, 30) in Richtung orthogonal zu der Symmetrieachse (18) des Schafts (12) abhängig von der eingestellten Blickrichtung (28, 38).

## Claims

1. **A device** (10) **for capturing a stereo image,** having:
a **first objective** (40) for producing a first image that is intended to be observed by a first eye of an observer;
a **second objective** (50) for producing a second image, that is intended to be observed by a second eye of an observer;
a **first viewing direction apparatus** (20), that is **rotatable** about a first axis of rotation (48) and assigned to the first objective (40);
a **second viewing direction apparatus** (30), that is **rotatable** about a second axis of rotation (58) and assigned to the second objective (50),
a **first image sensor** (64) for capturing the first image;
a **second image sensor** (74) for capturing the second image; and
a cam mechanism (49, 94) having a drive shaft (90);
wherein the viewing direction (28, 38) of the device (10) is rotatable by simultaneously rotating the first viewing direction apparatus (20) about the first axis of rotation (48) and the second viewing direction apparatus (30) about the second axis of rotation (58),
wherein the first objective (40) or a part of the first objective (40) is movable in a translational manner,
wherein the **cam mechanism** (49, 94) is formed to couple a rotation of the first viewing direction apparatus (20) to a translational movement of the first objective (40) or of part of the first objective (40),
and wherein the **drive shaft** (90) is rotationally coupled to the first viewing direction apparatus (20) and to the second viewing direction apparatus (30) and is arranged **between** the first objective (40) and the second objective (50).

2. The device (10) according to the preceding claim, in which the cam mechanism (49, 94) or a further cam mechanism (69, 96) is provided and formed to couple a rotation of the first viewing direction apparatus (20) to a translational **movement of the first viewing direction apparatus** (20) in the direction (48), in which the first objective (40) or a part of the first objective (40) is movable.

3. The device (10) according to one of the preceding claims, in which the cam mechanism or a further cam mechanism (69, 96) is provided and formed to couple a rotation of the first viewing direction apparatus (20) to a translational **movement of the first image sensor** (64) assigned to the first objective (40) or a light entry surface of an image transmission apparatus.

4. The device (10) according to one of the preceding claims, in which the first objective (40) comprises a first part and a second part, wherein the device is formed such that, either, only the translational movement of the first part is coupled to the rotation of the first viewing direction apparatus or that a rotation of the first viewing direction apparatus is accompanied by different translational movements of the first part and of the second part of the first objective (40).

5. The device (10) according to one of the preceding claims, in which the first viewing direction apparatus (20) and the second viewing direction apparatus (30) are each arranged at least partially next to the drive shaft (90).

6. The device (10) according to one of the preceding claims, in which the drive shaft (90) comprises a cam carrier, which is a constituent part of the cam mechanism, or said drive shaft is rotationally coupled to a cam carrier, which is a constituent part of the cam mechanism, a cam (94), which is a constituent part of the cam mechanism, is provided on the outer side of the cam carrier for coupling a rotation of the first viewing direction apparatus (20) to a translational movement of the first objective (40) or of part of the first objective (40).

7. The device (10) according to one of the preceding claims, in which the cam mechanism comprises a **groove** (94, 96) or a web or a step at the drive shaft (90) or at a cam carrier (92) that is rotationally coupled to the drive shaft (90) and a pin or a web or any other sensing device at the first objective (40) or a part of the first objective (40) or at a component (22) that is translationally coupled to the first objective (40) or a part of the first objective (40).

8. The device (10) according to one of the preceding claims without claim 7, in which the cam mechanism comprises a **groove** or a web or a step at the first objective (40) or a part of the first objective (40) or at a component (22) that is translationally coupled to the first objective (40) or a part of the first objective (40) and a sensing device at the drive shaft (90) or at a component (25) that is rotationally coupled to the drive shaft (90).

9. The device (10) according to one of the preceding claims, further having:
a **shaft** (12) with a distal end (14), wherein the outer lateral face (13) of the shaft (12) is **rotationally symmetric** in relation to an axis of symmetry (18) at least close to the distal end (14) of the shaft (12), wherein the first viewing direction apparatus (20), the second viewing direction apparatus (30), the first objective (40) and the second objective (50) are arranged in the shaft (12), wherein at least in one viewing direction (28) of the device (10), a proximal portion (48) of the optical axis of the first viewing direction apparatus (20) and a proximal portion (58) of the optical axis of the second viewing direction apparatus (30) are **not arranged mirror symmetrically** in relation to a plane of symmetry that contains the axis of symmetry (18) of the shaft (12).

10. The device (10) according to the preceding claim, further having: an apparatus for the translational movement of the viewing direction apparatuses (20, 30) in a direction orthogonal to the axis of symmetry (18) of the shaft (12), depending on the set viewing direction (28, 38).

## Revendications

1. **Dispositif** (10) **pour capturer une image stéréo,** ayant :
un **premier objectif** (40) pour produire une première image qui est destinée à être observée par un premier œil d'un observateur ;
un **second objectif** (50) pour produire une seconde image, qui est destinée à être observée par un second œil d'un observateur ;
un **premier appareil de direction de vision** (20), qui **peut être tourné** autour d'un premier axe de rotation (48) et affecté au premier objectif (40) ;
un **second appareil de direction de vision** (30), qui est **rotatif** autour d'un second axe de rotation (58) et affecté au second objectif (50),
un **premier capteur d'images** (64) pour capturer la première image ;
un **second capteur d'image** (74) pour capturer la seconde image ; et
un mécanisme à came (49, 94) ayant un arbre d'entraînement (90) ;
dans lequel la direction de vision (28, 38) du dispositif (10) peut être tournée en faisant tourner simultanément le premier appareil de direction de vision (20) autour du premier axe de rotation (48) et le second appareil de direction de vision (30) autour du second axe de rotation (58),
dans lequel le premier objectif (40) ou une partie du premier objectif (40) est mobile en translation,
dans lequel le **mécanisme à came** (49, 94) est formé pour coupler une rotation du premier appareil de direction de vision (20) à un mouvement de translation du premier objectif (40) ou d'une partie du premier objectif (40),
et dans lequel **l'arbre de transmission** (90) est couplé en rotation au premier appareil de direction de vision (20) et au second appareil de direction de vision (30) et est agencé **entre** le premier objectif (40) et le second objectif (50).

2. Dispositif (10) selon la revendication précédente, dans lequel le mécanisme à came (49, 94) ou un mécanisme à came supplémentaire (69, 96) est fourni et formé pour coupler une rotation du premier appareil de direction de vision (20) à un **mouvement** de translation **du premier appareil de direction de vision** (20) dans la direction (48), dans lequel le premier objectif (40) ou une partie du premier objectif (40) est mobile.

3. Dispositif (10) selon l'une des revendications précédentes, dans lequel le mécanisme à came ou un mécanisme à came supplémentaire (69, 96) est fourni et formé pour coupler une rotation du premier appareil de direction de vision (20) à un **mouvement** de translation **du premier capteur d'image** (64) affecté au premier objectif (40) ou à une surface d'entrée de lumière d'un appareil de transmission d'images.

4. Dispositif (10) selon l'une des revendications précédentes, dans lequel le premier objectif (40) comprend une première partie et une seconde partie, dans lequel le dispositif est formé de telle sorte que, soit, seul le mouvement de translation de la première partie est couplé à la rotation du premier appareil de direction de vision, soit une rotation du premier appareil de direction de vision est accompagnée de différents mouvements de translation de la première partie et de la seconde partie du premier objectif (40).

5. Dispositif (10) selon l'une des revendications précédentes, dans lequel le premier appareil de direction de vision (20) et le second appareil de direction de vision (30) sont chacun agencés au moins partiellement à côté de l'arbre d'entraînement (90).

6. Dispositif (10) selon l'une des revendications précédentes, dans lequel l'arbre d'entraînement (90) comprend un support de came, qui est une partie constitutive du mécanisme à came, ou ledit arbre d'entraînement est couplé en rotation à un support de came, qui est une partie constitutive du mécanisme à came, une came (94), qui est une partie constitutive du mécanisme à came, est fournie sur le côté extérieur du support de came pour coupler une rotation du premier appareil de direction de vision (20) à un mouvement de translation du premier objectif (40) ou d'une partie du premier objectif (40).

7. Dispositif (10) selon l'une des revendications précédentes, dans lequel le mécanisme à came comprend une **rainure** (94, 96) ou une bande ou un échelon au niveau de l'arbre d'entraînement (90) ou d'un support de came (92) qui est couplé en rotation à l'arbre d'entraînement (90) et une broche ou une bande ou tout autre dispositif de détection au niveau du premier objectif (40) ou d'une partie du premier objectif (40) ou au niveau d'un composant (22) qui est couplé en translation au premier objectif (40) ou à une partie du premier objectif (40).

8. Dispositif (10) selon l'une des revendications précédentes hormis la revendication 7, dans lequel le mécanisme à came comprend une **rainure** ou une bande ou un échelon au niveau du premier objectif (40) ou d'une partie du premier objectif (40) ou au niveau d'un composant (22) qui est couplé en translation au premier objectif (40) ou à une partie du premier objectif (40) et un dispositif de détection au niveau de l'arbre d'entraînement (90) ou d'un composant (25) qui est couplé en rotation à l'arbre d'entraînement (90).

9. Dispositif (10) selon l'une des revendications précédentes, ayant en outre: un **arbre** (12) avec une extrémité distale (14), dans lequel la face latérale extérieure (13) de l'arbre (12) est **symétrique en rotation** par rapport à un axe de symétrie (18) au moins à proximité de l'extrémité distale (14) de la tige (12), dans lequel le premier appareil de direction de vision (20), le second appareil de direction de vision (30), le premier objectif (40) et le second objectif (50) sont agencés dans la tige (12), dans lequel au moins dans une direction de vision (28) du dispositif (10), une partie proximale (48) de l'axe optique du premier appareil de direction de vision (20) et une partie proximale (58) de l'axe optique du second appareil de direction de vision (30) ne sont **pas agencés en symétrie spéculaire** par rapport à un plan de symétrie qui contient l'axe de symétrie (18) de l'arbre (12).

10. Dispositif (10) selon la revendication précédente, ayant en outre: un appareil pour le mouvement de translation des appareils de direction de vision (20, 30) dans une direction orthogonale à l'axe de symétrie (18) de l'arbre (12), en fonction de la direction de vision établie (28, 38).
